# EUROPEAN PATENT SPECIFICATION

(11) **EP 1 300 394 B1**
(45) Date of publication and mention of the grant of the patent: **11.02.2004**
(21) Application number: 00949487.3
(22) Date of filing: 13.07.2000
(51) Int. Cl.: C07C 403/24, A23L 1/275, A61K 47/06, A61P 3/02, A61P 39/06

(54) **METHOD FOR THE PRODUCTION OF A WATER-DISPERSIBLE FORMULATION CONTAINING CAROTENOIDS**
VERFAHREN FÜR DIE PRODUKTION EINER IN WASSER DISPERSIBLEN CAROTENOID-HALTIGEN FORMULIERUNG
PROCEDE DE PRODUCTION D'UNE FORMULATION SOLUBLE DANS L'EAU CONTENANT DES CAROTENOIDES

(43) Date of publication of application: 09.04.2003
(73) Proprietor: Vitatene, S.A., 24080 Leon (ES); Rudolf Wild GmbH & Co. KG, 69052 Heidelberg (DE)
(72) Inventor: ELLER, Thomas, 69052 Heidelberg (DE); CHRISTIANSEN, Christian, 69052 Heidelberg (DE); COLLADOS DE LA VIEJA, Alfonso, J., E-24080 León (ES); MUNOZ, Angel, E-24080 León (ES); ESTEBAN MORALES, Manuel, E-24080 León (ES)
(74) Representative: Elzaburu, Alberto de
(86) International application number: PCT/ES2000/000250
(87) International publication number: WO 2002/008182

(56) References cited:
- EP-A1- 0 937 412
- WO-A1-94/19411
- GB-A- 887 883
- US-A- 4 844 934
- US-A- 4 915 965
- US-A- 5 364 563
- US-A- 5 968 251

## Description

The present invention relates to health granulates containing carotenoids and a process for the production thereof. More particularly, the current invention relates to encapsulated carotenoids, especially β-carotene, obtained by utilising various kinds of nutritional starches and formulated in granules excellent in handling properties, compressibility, colour release and appearance, and useful in colouring beverages, formulation of solids and semisolids pharmaceutical preparation and promoting fish growth.

### 2. Prior Art

It is public knowledge in the art that natural β-carotene is utilised in several forms as natural colouring agents for food, cosmetics, fish feed and the like as additives for health food and as a material for regulating vital functions such as a nourishing diet supplement. However, no practical proposal had been made concerning the method of fully health-oriented granulate, i.e., without halogenated solvents or animal proteins.

Among these carotenoids, β-carotene, one of provitamin A group compounds, is easily oxidizable because it contains a conjugated double bond chains and also a poorly watersoluble compound. These are serious problems in the food industry and especially in the beverage manufacture in which drinks are almost a hundred percent water-based products. Furthermore demanding on health food, mainly from natural sources, is one of the main tasks of Food and Drug Administrations. It has therefore been strongly desired to develop a method by which the carotenoids can be processed without destroying the natural carotene, and the product obtained can be also be preserved in a stable state and in soluble form.

Solubility problem was mentioned firstly in US3998753 and solved by preparing carotenoids solutions in volatile organic solvents such halogenated hydrocarbons as and emulsifying with a water solution of sodium lauril sulphate. A similar description as found in EP937412 by using a continuous process. These processes, however, involved the use of chloride hydrocarbons, which are well known as toxic products with a limited use in pharmaceutical and food industries, but also environmental risks are derived from the residual solvents.

A proposal to eliminate these solvents was found in ES2022470, in this carotenoids are added as water dispersable powder, in this process carotenoids were solubilized previously in a food-quality oil. In this formulation the carotenoids is formulated by adding numerous ingredients (stabilisers, emulsifiers, etc.). Thus, this was not a natural product as is demanded by consumers. US5364563 describes a process for producing a powdered carotenoid preparation comprising forming a suspension of a carotenoid in high boiling oil without the presence of an organic solvent. The suspension is superheated with a steam for a maximum period of thirty seconds to form a solution of carotenoid in oil. After that, solution is emulsified in an aqueous solution of a colloid and thereafter spraying and drying the emulsion a powder is formed. However, great losses of carotenoids at the high temperatures used during the treatment with superheated steam and also some isomerizations occur. WO93/04598 proposed the use of natural additives, such as, stabilisers, acidifiers and emulsifiers. US5714658 described the use of a mixture of different solvents including esters of acetic acid, alcohol C1-C4 and oil compatibles with the other solvents. In this process, two problems are involved: firstly, the low solubility of carotenoids in the mixture of solvents, which implies a large amount of oil and solvents required, especially if a high concentration of carotenoids is demanded, and secondly the environmental risks derived from the residual solvents. In EP807431 is described a procedure to obtain a water solution of carotenoids with a protective colloid, at high temperature (180-250°C) and pressure (96.6-2759 bar) a emulsion is obtained which after drying yield the carotenoid in form of dry powder. However, the equipments and conditions required result in a very expensive process. US5780056 with priority number JP1996000141034 describes microcapsules which are made of a core material comprising natural carotenoid and an edible oil, and a wall which is made of a coating material based on gelatin, the particles have a mean particle size of 0.01-5 µm. The microcapsule has a strength enough to protect natural carotenoid from oxidation and deterioration for a long time and to withstand tableting pressure. The colloid generally used is gelatin originating from warm-blooded animals and such origin is often problematic. For example, preparations based on such gelatin can not be used worldwide for religious reasons.

In accordance with US5478569 all of these disadvantages can be eliminated when fish gelatin is used in place of gelatin from warm-blooded animals. Also without an expensive manufacturing process of this gelatin, the resulting manufactured preparations do not always have a desired dispersibility in cold water, etc. Furthermore, problems derived by using colloids as gelatine and collagen from animal source are related to the capability of these proteins to be carrier of infections and more particularly of the crazy cow illness.

In order to obtain greatest benefit from the carotenoids, the particles should either dissolve rapidly or be sufficiently wettable to be easily absorbed. As above-described the first strategy was to manufacture microcapsules as US5478569 and US5780056. However, if a large amount of edible oil is contained for dispersing natural carotenoid, the resulting microcapsules are reduced in strength and the stabilizing effect of gelatin against oxidation as a protective coating is not fully exerted. Natural carotenoid is then vulnerable to oxidation and deterioration. Low microcapsule strength has another problem. When microcapsules are blended with a vehicle and compressed into tablets, the microcapsules can be broken during tableting, allowing carotenoid to leak out. It is thus difficult to blend carotenoid in tablets in a stable manner.

The second strategy US3907983 could be spray-coated from aqueous solutions with from about 0.5% to about 10% by weight of a hydrophilising material selected from the group consisting of mtehylcellulose, hydroxyethylmethylcellulose and hydroxypropylmethylcellulose. The limitation consists that procedure by which the hydrophilising material is applied to the surface of the individual particles must be carried out in a manner which insures that the particles do not become agglomerated.

The last strategy deals with granulation process. Thus, US4915966 (priority numbers JP1988000040754 and JP 1988000040755) granulated dried powder of Dunaliella algae, which is rich in β-carotene, with other materials to make a granulation. The way to process without destroying the natural β-carotene, and preserved in a stable state was by adding cyclodextrin to dried powder of Dunaliella algae in a certain ratio. Granulation was encapsulated in a hard capsule. β-cyclodextrins was used also in WO96/40262 for stabilising β-carotene in a water soluble organic solvent. Additional important improvement, also recently found in EP 8325692A2, was the use of only organic solvent without halogens and in a small extent.

The present inventors actively investigated possible solutions for the aforementioned problems, especially those related to use of colloids from animal sources. As a result of such investigation, they have completed the present invention by the development of a process in which the natural β-carotene is preserved in a stable state by food-grade starch matrix. Only US3790688 was a previous attempt to formulate β-carotene by using starch, but in this case a precipitate by solvent addition was done instead the emulsion of the process hereby described. The granulation makes the natural β-carotene hydrophilic, dispersible and bioavailable, and therefore acceptable as nutritional supplement in foods. The resulted powder is free flowing as well as amenable to tabletting by direct compression or encapsulated in a hard-capsule are disclosed as well as a method for making such granulation and formulations.

### SUMMARY OF THE INVENTION

It is an object of the present to provide water-dispersible carotenoids formulation containing wherein from about 1 to 25 parts by weight of dried powder of carotenoid encapsulated in a matrix of food-quality starch which provides β-carotene included in the encapsulated powder in a stable condition. The formulations containing natural carotenoids having satisfactory physiological activity to the human, body and a high strength enough to prevent the carotenoid from being oxidized or deteriorated during long-term storage. The formulations are suitable to be blended in capsules or tablets or the like in a stable manner, and in such applications as additive agents for foods and pharmaceuticals.

It is another object of this invention to provide a process for producing the formulation which comprises the following steps: adding β-carotene to mixture of natural or synthetic tocopherols in vegetable oil and dissolving in n-butil acetate or iso-butyl acetate, dissolving a mixture of two different starches in desionized water, preparing a emulsion by admixing both phases and eliminating organic solvent under temperatures between 50-80° and reduced pressure until 15-25% of dry matter is obtained, drying to obtain final product.

In accordance with the present invention it has been found that both the spray-drying method and, especially fluidised bed method are suitable.

According to the spray-drying method, the inlet temperature was between 100-190°C, while outlet temperature was 80-150°C.

According to the fluidised-bed method. The seed material, i.e. typical inert material as sugar particles, as well as dust or fine powder obtained from previous granulation processes or from spray-dried process are placed in a fluidized-bed granulator. Particles are maintained in motion by means of air, the input and output temperature of which is adjusted according between 30-90°C. A solution of the liquid formulation (15-25% of dry matter) is sprayed into the drier at a rate, which ensures that the particles to be coated do not become too damp and conglomerate. After completion of the spraying, the particles can be coated.

The invention is also concerning with the spray-coating from about 0.5% to about 10% by weight aqueous contained sugar solutions or from of a hydrophilising material selected, i. e. methylcellulose, hydroxyethylmethylcellulose and hydroxypropylmethylcellulose. Particles can be dried directly by a further input of suitably prewarmed air.

It is a further object of the invention to provide a hard capsule containing granulate which comprises the following, step: blending dried powder of granulate with fillers and/or lubricants and encapsulating in the light-impermeable hard capsules, thereby keeping the β-carotene included in the encapsulated form in a stable condition.

The invention is also concerned with pharmaceutical preparations in a powdered form or in the form of tablets containing carotenoid granulation in combination with conventional pharmaceutically acceptable tabletting adjuvants and/or excipients. Suitable pharmaceutically acceptable tabletting adjuvants and excipients are micro-crystalline cellulose, dextrose, lactose, sucrose, mannitol, glucose, sorbitol; lubricants such as calcium stearate, stearic acid or magnesium stearate as well as mixtures thereof. Talc, cornstarch etc. can be mixed with the lubricants. Flavoring agents and coloring agents can also be used. The process for producing tablets containing β-carotene comprises the following steps: blending and/or granulating the watter-dispersible formulation with adjuvants and/or excipients and tabletting in an eccentric or rotary tablet press at applied pressure between 30 and 200 MPa. The β-carotene remained in the starch matrix after compression in a stable condition and release β-carotene in a very short time.

### DETAILED DESCRIPTION OF THE INVENTION

The products of the present invention are water-dispersible solid formulations of β-carotene. The term water dispersible means that the formulations of the present invention shows UV-visible spectrum characteristic of β-carotene after dissolution and filtration. Because of the interaction of light with very small particles crystals appear the more yellow the smaller they become. β-carotene crystals of about 3-5 µm size are tomato red. Color changes for orange if particle size is about 1 µm and the dispersion appear yellow if the particle diameter becomes as small as about 0.6 µm. This effect is caused by shifts in the absorption spectrum of light.

### β-CAROTENE

β-carotene is a naturally occurring precursor of Vitamin A and is often used as an important colour for the beverage industry. β-carotene is typically derived by extraction from fermentation sources or synthesised using known chemical process. However, β-carotene is easily degraded when subjected to air, light or temperatures. Thus, β-carotene is stabilised and available in this form from, for example, BASF Corporation or Hoffman LaRoche. In the present invention, it is preferred to use β-carotene crystals.

The β-carotene is dissolved in an organic solvent. The β-carotene have been suspended in Iso-Butyl Acetate (IBA). The suspension has been milled in a ball mill (Netzch minizetal) at 1500 rpm for about 10-15 minutes. The viscous paste has been transferred into warm and continuously stirred IBA at 60°C. The IBA suspension has been rapidly heated to 105°C. The temperature was hold for 5 min. Afterwards the solution of β-carotene is formed and cool down to 85-90°C.

The composition of the present invention where from about 1 to 25 part of β-carotene by weight of dried powder, the remainder being OSA-starches.

### STARCHES

The starches applied are Purity Gum 2000, modified food starch from National Starch & Chemical, this permits easy emulsification of the organic phase in water. After solvent removal the dispersion meets the color shade of β-carotene. This modified food starch does not give enough redispersability of dried powder. Thus, another starch is also added.

The second variety of food grade starch is Hi-Cap 100 (National Starch & Chemical), this provides a good dispersability of the final product.

### ADDITIONAL INGREDIENTS

Because β-carotene is sensible to oxidation, antioxidants may be dissolved in the solvent containing the β-carotene to enhance the stability against deterioration. Any antioxidant approved for food may be used in the present invention, including but not limited to α-tocopherol from natural or synthetic source. The level of antioxidant would be sufficient for β-carotene protection. This should be from 0.1 to 0.3 times the amount of β-carotene. Ascorbyl palmitate can be also added to the formulation due to the synergistic antioxidative effect of the combination of both antioxidants.

### METHOD OF PREPARATION

After dissolving β-carotene in Iso Butyl Acetate the next step is the emulsification. In this, the solution of β-carotene with antioxidants in the mentioned solvent at 85-90°C is poured into the starch solution in water. The dark brown solution has a temperature below 50°C and has immediately afterwards homogeneized by a rotor-stator system (IKA MF45) for 10 min. The emulsion became dark orange within the first minute.

The solvent evaporation is at 40-45°C and reduced pressure (approx. 65 mbar) within 30 minutes. Solvent residues is removed with the water evaporated during several hours.

For obtaining the solid formulation the preferred method is the fluidized-bed. In this, the seed material, i.e. typical inert material as sugar particles, as well as dust or fine powder obtained from previous granulation processes or from spray-dried process are placed in a fluidized-bed granulator. Particles are maintained in motion by means of air, the input and output temperature of which is adjusted according between 30-90°C. A solution of the liquid formulation (15-25% of dry matter) is sprayed into the drier at a rate, which ensures that the particles to be coated do not become too damp and conglomerate.

### EXAMPLE 1

20g of crystalline β-carotene have been suspended together with 3g of D-tocopherol extract (Nutrilo) in 800 mL n-butyl acetate. The suspension has been refluxed for 15 min at 130°C. A mixture of 90g Hi-Cap 100 and 40g Purity Gum 2000 (National Starch) has been dissolved in 650g of cation exchange water. The hot organic phase was emulsified in one step in the aqueous phase using an Ultraturrax (IKA). The dispersion has been diluted with 2 L of water. Together with 800 mL of n-butyl acetate these two liter of water has been evaporated from 5 mL flask of a rotatory Evaporator (Büchi). The flask has been heated to about 80°C and the pressure gradient was as rapid as possible from 1024 mbar down to 60 mbar. Three times 1 L of water has been added and again evaporated to remove all the traces of solvent. 776g of dispersion (19.1 % of dry mass) with a β-carotene content of 2.4% in solution (12.7% β-carotene referred to dry mass) is obtained. The dispersion is granulated and dried in fluidized bed (UniGlatt) of sugar crystals. A free-flowing powder water-wettable and very fast dispersible was obtained.

**Table 1:**

| Ingredients of β-carotene formulation. | | |
|---|---|---|
| Dry mass (%) | Typical quantity | Ingredients |
| 58.8% | 90 g | Hi-Cap 100 |
| 26.1% | 40 g | Purity Gum 2000 |
| 13.1% | 20 g | Crystalline β-carotene |
| 2.0% | 3 g | Em 70 (nutrilo) |
| | 650 g | Cation exchanged water |
| | 800 mL | n-butyl acetate |

### EXAMPLE 2

396 g of crystalline β-carotene have been suspended together with 3.96 g of synthetic α-tocopherol (Roche) in 800 mL isobutyl acetate. The suspension has been milled in a Netzsch Mini-Zeta (Glass beads, 0.8-1.2 mm) at 1500 rpm for about 10-15 min. The viscous paste has completely transferred into warm and continuously stirred Iso-Butyl Acetate (60°C). The total amount of Iso-Butyl Acetate was 15840 g. The IBA suspension has been rapidly heated to 105°C. The temperature was hold for 5 min. Afterwards the solution of β-carotene is formed and cool down to 85-90°C within the next 10 min. A mixture of 1287 g Hi-Cap 100 and 1287 g Puritv Gum 2000 (National Starch) has been dissolved in 12870g of cation exchange water. The hot organic phase was emulsified in one step in the aqueous phase using a rotor-stator system (IKA MF45). The dispersion has been diluted with water and Iso-butyl acetate has been rapidly evaporated within 30 min. at 40-45°C at 65 mbar. Solvent residues have been removed with the water evaporated during several hours. The dispersion is granulated and dried in continuous fluidized bed granulator AGT 150 (Glatt) without seed material. A free-flowing powder water-wettable and very fast dispersible was obtained.

**Table 2:**

| Ingredients of β-carotene formulation. | | |
|---|---|---|
| Dry mass (%) | Typical quantity | Ingredients |
| 42.76% | 1287 g | Hi-Cap 100 |
| 42.76% | 1287 g | Purity Gum 2000 |
| 13.16% | 396 g | Crystalline β-carotene |
| 1.31% | 39,6 g | Synthetic α-tocopherol (Roche) |
| | 12870 g | Cation exchanged water |
| | 15840 Ml | iso-butyl acetate |

## Claims

1. A process for producing a water-dispersible granulated carotenoid preparation which comprises forming a suspension of carotenoid in insoluble or poorly watersoluble, non-halogenated, solvent and flavour compounds, with an antioxidant, warming up said suspension to form a solution of said carotenoid in said solvent, emulsifying said solution in an aqueous solution of a mixture of at least 2 different food-grade starch colloids, one with improved emulsifying effect and the other with improved dispersion effect, removing the solvent, concentrating the liquid emulsion formed thereof and thereafter drying said emulsion until obtaining the desired granulate.

2. Process according to claim 1 wherein said carotenoid is β-carotene.

3. Process according to any of the claims 1 or 2 wherein said solvent is n-butyl acetate.

4. Process according to any of the claims 1 or 2 wherein said solvent is iso-butyl acetate.

5. Process according to any of the claims 1 or 2 wherein said solvent is limonene.

6. Process according to any of the claims 1 to 5 wherein the temperature to form the solution reaches a maximum of 130°C.

7. Process according any of the claims 1 to 6 wherein the warming temperature for removing the solvent and concentrating the liquid emulsion reaches a maximum of 80°C.

8. Process according to claim 6 wherein the removal of solvent and the concentration of the liquid emulsion is made under vacuum by forming an azeotropic mixture with water.

9. Process according to any of the claims 1 to 7 wherein the liquid emulsion is dried by conventional spray-drying means.

10. Process according to any of the claims 1 to 8 wherein the liquid emulsion is dried by fluid-bed means.

11. A product which consists of a granulate of carotenoids in amorphous or microcrystalline form, obtainable by the process of any of the claims 1 to 10 **characterized by** consisting in a multi-layer structure comprising a central-core, covered with a matrix of carotenoid based on a mixture of at least 2 different food-grade starch colloids, one with improved emulsifying effect and the other with improved dispersion effect, wherein content of carotenoid was from 1 to 25 parts by weight of granulate, said particles having a mean particle size of 0.5 to 3 mm and wherein water content is up to 10% by weight based on the weight of said granulate.

12. The product of claim 11 which consists in a free-flowing and water dispersible granulate directly compressible into rapidly absorbed tablets or capsules.

13. The product of claims 11-12 wherein the central-core is made of a sugar.

14. The product of claims 11-12 wherein the central-core is the matrix of carotenoid itself based on food-grade starch.

15. The product of claims 11-14 wherein the carotenoid is β-carotene.

16. The product of claims 11-15 spray-coated with 0.5% to 10% by weight of aqueous saturated sugar solutions.

17. The product of claims 11-15 spray-coated with 1% to 5% by weight of a hydrophilising material, selected among else from methylcellulose, hydroxyethylmethylcellulose or hydroxypropylmethylcellulose.

18. An additive or colouring agent for food, beverages, nutrition fortified food and pharmaceuticals comprising granulates as set forth in claims 11-17.

19. A tablet comprising product as set forth in claims 11-17.

20. A tablet according to claim 19 further comprising adjuvants/excipients.

21. A tablet according to claim 20, wherein said adjuvants/excipients are selected among else from a group of micro-crystalline cellulose, dextrose, lactose, sucrose, mannitol, glucose, sorbitol; lubricants such as calcium stearate, stearic acid or magnesium stearate, talc, cornstarch as well as mixtures thereof.

22. A tablet according to any of the claims 20 to 21, wherein said tablet contains 0.1 to 30% by weight of the product according to claims 11-17.

23. A hard-capsule comprising a product as set forth in claims 11-17 and adjuvants/excipients.

24. A hard-capsule according to claim 23, wherein said adjuvants/excipients are selected among else from a group of, dextrose, lactose, sucrose, mannitol, glucose, sorbitol; lubricants such as talc, calcium stearate, stearic acid or magnesium stearate as well as mixtures thereof.

25. A hard-capsule according to any of the claims 23 to 24, wherein said hard-capsule contains 0.1 to 30% by weight of granulates.

## Patentansprüche

1. Verfahren zur Herstellung einer wasserdispergierbaren granulierten Carotinoid-Zubereitung, umfassend die Bildung einer Suspension von Carotinoid in einem wasserunlöslichen oder schwer wasserlöslichen, nicht-halogenierten Lösungsmittel und Geschmackstoffen mit einem Antioxidans, Erwärmung der Suspension, um eine Lösung des Carotinoids in dem Lösungsmittel zu bilden, Emulgieren der Lösung in einer wässrigen Lösung eines Gemisches von mindestens 2 verschiedenen nahrungsmittelgeeigneten Stärkekolloiden, eines mit verbessertem emulgierenden Effekt und das andere mit verbessertem Dispersionseffekt, Entfernung des Lösungsmittel, Konzentrierung der davon gebildeten Flüssigemulsion, und danach Trocknung der Emulsion bis man das gewünschte Granulat erhält.

2. Verfahren gemäß Anspruch 1, wobei das Carotinoid β-Carotin ist.

3. Verfahren gemäß einem der Ansprüche 1 oder 2, wobei das Lösungsmittel n-Butylacetat ist.

4. Verfahren gemäß einem der Ansprüche 1 oder 2, wobei das Lösungsmittel iso-Butylacetat ist.

5. Verfahren gemäß einem der Ansprüche 1 oder 2, wobei das Lösungsmittel Limonen ist.

6. Verfahren gemäß einem der Ansprüche 1 bis 5, wobei die Temperatur, um eine Lösung zu bilden, ein Maximum von 130° C erreicht.

7. Verfahren gemäß einem der Ansprüche 1 bis 6, wobei die Erwärmungstemperatur zur Entfernung des Lösungsmittels und zur Konzentrierung der Flüssigemulsion ein Maximum von 80° C erreicht.

8. Verfahren gemäß Anspruch 6, wobei das Entfernen des Lösungsmittels und die Konzentrierung der Flüssigemulsion unter Vakuum durch Bildung eines azeotropen Gemisches mit Wasser durchgeführt wird.

9. Verfahren gemäß einem der Ansprüche 1 bis 7, wobei die Flüssigemulsion mit herkömmlichen Sprühtrocknungsvorrichtungen getrocknet wird.

10. Verfahren gemäß einem der Ansprüche 1 bis 8, wobei die Flüssigemulsion mit einer Wirbelbettvorrichtung getrocknet wird.

11. Produkt, das aus einem Granulat von Carotinoiden in amorpher oder mikrokristalliner Form besteht, erhältlich mit einem Verfahren nach einem der Ansprüche 1 bis 10, **gekennzeichnet durch** das Bestehen in einer mehrschichtigen Struktur, die einen Zentralkern umfasst, umhüllt mit einer Carotinoid-Matrix, basierend auf einem Gemisch von mindestens 2 verschiedenen nahrungsmittelgeeigneten Stärkekolloiden, eines mit verbessertem emulgierenden Effekt und das andere mit verbessertem Dispersionseffekt, wobei der Carotinoidgehalt 1 bis 25 Gewichtsteile des Granulates betrug, wobei die Teilchen eine mittlere Partikelgröße von 0,5 bis 3 mm besitzen und wobei der Wassergehalt bis zu 10 Gew.-%, bezogen auf das Granulatgewicht ist.

12. Produkt nach Anspruch 11, das aus einem rieselfähigen und wasserdispergierbaren Granulat besteht, das direkt in schnell resorbierte Tabletten oder Kapseln komprimierbar ist.

13. Produkt nach Ansprüchen 11-12, wobei der Zentralkern aus Zucker gemacht ist.

14. Produkt nach Ansprüche 11-12, wobei der Zentralkern die Carotinoid-Matrix selbst ist, basierend auf nahrungsmittelgeeigneter Stärke.

15. Produkt nach Ansprüche 11-14, wobei das Carotinoid β-Carotin ist.

16. Produkt nach Ansprüche 11-15, das mit 0,5 bis 10 Gew.-% wässriger gesättigter Zuckerlösungen sprühbeschichtet ist.

17. Produkt nach Ansprüche 11-15, das mit 1 bis 5 Gew.-% eines hydrophilisierenden Materials, ausgewählt unter anderem aus Methylcellulose, Hydroxyethylmethylcellulose oder Hydroxypropylmethylcellulose, sprühbeschichtet ist.

18. Zusatzstoff oder Farbstoff für Lebensmittel, Getränke, nährstoffangereicherte Lebensmittel und Arzneimittel, die Granulate wie in den Ansprüchen 11-17 dargelegt umfassen.

19. Tablette, die das Produkt wie in den Ansprüchen 11-17 dargelegt umfasst.

20. Tablette gemäß Anspruch 19, die ferner Hilfsstoffe/Exzipienten umfasst.

21. Tablette gemäß Anspruch 20, wobei die Hilfsstoffe/Exzipienten ausgewählt sind unter anderem aus mikrokristalliner Cellulose, Dextrose, Laktose, Saccharose, Mannitol, Glucose, Sorbit; Gleitmittel, wie zum Beispiel Calciumstearat, Stearinsäure oder Magnesiumstearat, Talk, Maisstärke ebenso wie Gemische davon.

22. Tablette gemäß einem der Ansprüche 20 bis 21, wobei die Tablette 0,1 bis 30 Gew.-% des Produkts gemäß den Ansprüchen 11-17 enthält.

23. Hartkapsel, die ein Produkt wie in den Ansprüchen 11-17 dargelegt und Hilfsstoffe/Exzipienten umfasst.

24. Hartkapsel gemäß Anspruch 23, wobei die Hilfsstoffe/Exzipienten ausgewählt sind unter anderem aus Dextrose, Laktose, Saccharose, Mannitol, Glukose, Sorbit; Gleitmittel, wie zum Beispiel Talk, Calciumstearat, Stearinsäure oder Magnesiumstearat ebenso wie Gemische davon.

25. Hartkapsel gemäß einem der Ansprüche 23 bis 24, wobei die Hartkapsel 0,1 bis 30 Gew.-% des Granulats enthält.

## Revendications

1. Procédé de production d'une préparation granulée de caroténoïde, dispersable dans l'eau, qui comprend la formation d'une suspension de caroténoïde dans un solvant non-halogéné, insoluble ou faiblement soluble dans l'eau, contenant des arômes et un antioxydant, le chauffage de ladite suspension pour former une solution dudit caroténoïde dans ledit solvant, l'émulsification de ladite solution dans une solution aqueuse d'un mélange d'au moins deux colloïdes différents d'amidon de qualité alimentaire, dont l'un a un effet émulsifiant accru et l'autre a un effet de dispersion accru, l'élimination du solvant, la concentration de l'émulsion liquide ainsi formée et ensuite le séchage de ladite émulsion jusqu'à l'obtention du granulé désiré.

2. Procédé selon la revendication 1, dans lequel ledit caroténoïde est le β-carotène.

3. Procédé selon la revendication 1 ou 2, dans lequel ledit solvant est l'acétate de n-butyle.

4. Procédé selon la revendication 1 ou 2, dans lequel ledit solvant est l'acétate d'isobutyle.

5. Procédé selon la revendication 1 ou 2, dans lequel ledit solvant est le limonène.

6. Procédé selon l'une quelconque des revendications 1 à 5, dans lequel la température pour former la solution atteint au maximum 130 °C.

7. Procédé selon l'une quelconque des revendications 1 à 6, dans lequel la température de chauffage pour éliminer le solvant et concentrer l'émulsion liquide atteint au maximum 80 °C.

8. Procédé selon la revendication 6, dans lequel l'élimination du solvant et la concentration de l'émulsion liquide sont réalisées sous vide, par formation d'un mélange azéotrope avec de l'eau.

9. Procédé selon l'une quelconque des revendications 1 à 7, dans lequel l'émulsion liquide est séchée par des moyens classiques de séchage par pulvérisation.

10. Procédé selon l'une quelconque des revendications 1 à 8, dans lequel on sèche l'émulsion liquide au moyen d'un lit fluidisé.

11. Produit constitué d'un granulé de caroténoïdes sous forme amorphe ou microcristalline, que l'on peut obtenir par le procédé selon l'une quelconque des revendications 1 à 10, produit **caractérisé en ce qu'**il a une structure multicouche comprenant un noyau central, recouvert d'une matrice de caroténoïde à base d'un mélange d'au moins deux colloïdes différents d'amidon de qualité alimentaire, dont l'un a un effet émulsifiant accru et l'autre a un effet de dispersion accru, la teneur en caroténoïde du granulé étant de 1 à 25 % en poids, lesdites particules ayant une taille moyenne de particule de 0,5 à 3 mm, et la teneur en eau étant au plus de 10 % en poids par rapport au poids dudit granulé.

12. Produit selon la revendication 11, qui est constitué d'un granulé s'écoulant librement et dispersable dans l'eau, que l'on peut comprimer directement en comprimés ou capsules rapidement absorbés.

13. Produit selon la revendication 11 ou 12, dont le noyau central est constitué d'un sucre.

14. Produit selon la revendication 11 ou 12, dont le noyau central est la matrice de caroténoïde elle-même, à base d'amidon de qualité alimentaire.

15. Produit selon l'une quelconque des revendications 11 à 14, pour lequel le caroténoïde est le β-carotène.

16. Produit selon l'une quelconque des revendications 11 à 15, qui est revêtu par pulvérisation de 0,5 % à 10 % en poids d'une solution aqueuse saturée de sucre.

17. Produit selon l'une quelconque des revendications 11 à 15, qui est revêtu par pulvérisation de 1 % à 5 % en poids d'un produit hydrophile, choisi parmi la méthylcellulose, l'hydroxyéthylméthylcellulose et l'hydroxypropylméthylcellulose, entre autres.

18. Additif ou agent colorant pour les aliments, les boissons, les aliments nutritionnels fortifiants et les produits pharmaceutiques, qui comprend des granulés tels que définis dans l'une quelconque des revendications 11 à 17.

19. Comprimé comprenant un produit tel que défini dans l'une quelconque des revendications 11 à 17.

20. Comprimé selon la revendication 19, qui comprend en outre des adjuvants/excipients.

21. Comprimé selon la revendication 20, pour lequel lesdits adjuvants/excipients sont choisis parmi la cellulose microcristalline, le dextrose, le lactose, le sucrose, le mannitol, le glucose, le sorbitol, des lubrifiants tels que le stéarate de calcium, l'acide stéarique et le stéarate de magnésium, le talc, l'amidon de maïs et leurs mélanges, entre autres.

22. Comprimé selon la revendication 20 ou 21, qui contient 0,1 à 30 % en poids de produit selon l'une quelconque des revendications 11 à 17.

23. Capsule dure qui comprend un produit tel que défini dans l'une quelconque des revendications 11 à 17, et des adjuvants/excipients.

24. Capsule dure selon la revendication 23, pour laquelle lesdits adjuvants/excipients sont choisis parmi le dextrose, le lactose, le sucrose, le mannitol, le glucose, le sorbitol, des lubrifiants tels que le talc, le stéarate de calcium, l'acide stéarique et le stéarate de magnésium, et leurs mélanges, entre autres.

25. Capsule dure selon la revendication 23 ou 24, qui contient 0,1 à 30 % en poids de granulés.
